(19) Europäisches Patentamt / European Patent Office / Office européen des brevets

(11) EP 3 612 834 B1

(12) FASCICULE DE BREVET EUROPEEN

(45) Date de publication et mention de la délivrance du brevet:
**04.12.2024 Bulletin 2024/49**

(21) Numéro de dépôt: **18720769.1**

(22) Date de dépôt: **17.04.2018**

(51) Classification Internationale des Brevets (IPC):
*G01N 33/00* (2006.01)

(52) Classification Coopérative des Brevets (CPC):
**G01N 33/0075;** G06F 30/13

(86) Numéro de dépôt international:
**PCT/FR2018/050966**

(87) Numéro de publication internationale:
**WO 2018/193205 (25.10.2018 Gazette 2018/43)**

(54) **OPTIMISATION DE LA DISTRIBUTION SPATIALE DE MOYENS DE MESURE DE LA QUALITE DE L'AIR**

OPTIMIERUNG DER RÄUMLICHEN VERTEILUNG VON LUFTQUALITÄTSMESSMITTELN

OPTIMIZATION OF THE SPATIAL DISTRIBUTION OF AIR QUALITY MEASUREMENT MEANS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.04.2017 FR 1753393**

(43) Date de publication de la demande:
**26.02.2020 Bulletin 2020/09**

(73) Titulaire: **Elichens**
**38000 Grenoble (FR)**

(72) Inventeur: **ZANINI, Paolo**
**38100 Grenoble (FR)**

(74) Mandataire: **INNOV-GROUP**
**209 Avenue Berthelot**
**69007 Lyon (FR)**

(56) Documents cités:
• **W.F. CASELTON ET AL: "Optimal monitoring network designs", STATISTICS & PROBABILITY LETTERS, vol. 2, no. 4, 1 August 1984 (1984-08-01), AMSTERDAM, NL, pages 223 - 227, XP055418674, ISSN: 0167-7152, DOI: 10.1016/0167-7152(84)90020-8**

• **WEI YI ET AL: "A Survey of Wireless Sensor Network Based Air Pollution Monitoring Systems", SENSORS, vol. 15, no. 12, 12 December 2015 (2015-12-12), pages 31392 - 31427, XP055418903, DOI: 10.3390/s151229859**

• **AHMED BOUBRIMA ET AL: "Optimal Deployment of Wireless Sensor Networks for Air Pollution Monitoring", 2015 24TH INTERNATIONAL CONFERENCE ON COMPUTER COMMUNICATION AND NETWORKS (ICCCN), 1 August 2015 (2015-08-01), pages 1 - 7, XP055418146, ISBN: 978-1-4799-9964-4, DOI: 10.1109/ICCCN.2015.7288443**

• **MURTY R N ET AL: "CitySense: An Urban-Scale Wireless Sensor Network and Testbed", TECHNOLOGIES FOR HOMELAND SECURITY, 2008 IEEE CONFERENCE ON, IEEE, PISCATAWAY, NJ, USA, 12 May 2008 (2008-05-12), pages 583 - 588, XP031266662, ISBN: 978-1-4244-1977-7**

• **DONALD J. CHMIELEWSKI ET AL: "On the theory of optimal sensor placement", AI CH E JOURNAL, vol. 48, no. 5, 1 May 2002 (2002-05-01), US, pages 1001 - 1012, XP055419094, ISSN: 0001-1541, DOI: 10.1002/aic.690480510**

## Description

### Domaine technique

L'invention concerne un système de mesure d'au moins une grandeur physique représentative de la qualité de l'air dans une zone d'observation.

L'invention trouve notamment son application dans la surveillance et le contrôle de la qualité de l'air dans des zones urbaines.

### Etat de la technique antérieure

Un système de mesure connu de l'état de la technique, notamment du document « Guidelines for Ambient Air Quality Monitoring » établi par le CPCB (Central Pollution Control Board) du ministère de l'environnement du gouvernement de l'Inde, daté d'avril 2003, (ci-après D1) comporte un ensemble de stations de mesure de la qualité de l'air. D1 mentionne (cf. §4.2.2) des critères pour sélectionner les emplacements des stations de mesure dans la zone d'observation. Toutefois, D1 précise (cf. §4.2.2.a) qu'il est difficile de savoir à l'avance si les emplacements sélectionnés vont refléter et rendre compte de la qualité de l'air dans la zone d'observation. D1 propose (cf. §4.2.2.a) alors d'effectuer des mesures préalables afin de vérifier la pertinence des emplacements.

Un tel système de mesure de l'état de la technique n'est pas entièrement satisfaisant, en particulier lorsque l'on souhaite augmenter la résolution spatiale des mesures. La solution proposée par D1 est très fastidieuse et coûteuse lorsque les stations de mesure présentent une densité spatiale élevée dans la zone d'observation.

Un procédé pour l'emplacement optimal des capteurs est connu de "Optimal Monitoring Network design" (Caselton and Zidek, Statistics & Probability Letters 2 (1984) 223-227) qui décrit le choix optimal de l'emplacement des capteurs parmi toutes les positions possibles pour le placement des capteurs en minimisant l'incertitude pour les valeurs aux positions non choisies.

### Exposé de l'invention

L'invention vise à remédier en tout ou partie aux inconvénients précités. A cet effet, l'invention a pour objet un système de mesure d'au moins une grandeur physique représentative de la qualité de l'air dans une zone d'observation, le système comportant :

- une cartographie de la zone d'observation, comprenant un ensemble, noté V, de valeurs modélisées représentatives de la grandeur physique ;
- des moyens de mesure de la grandeur physique, possédant un nombre N de positions ou un nombre N de trajectoires dans la zone d'observation, les N positions ou les N trajectoires étant destinées à présenter une distribution spatiale, notée $S_{opt}$ , dans la zone d'observation ;
- des moyens de calcul de la distribution spatiale $S_{opt}$, configurés pour :

  - effectuer un maillage de la zone d'observation, le maillage comprenant un nombre G de points ;
  - calculer, pour une distribution spatiale donnée des N positions ou des N trajectoires, notée S, un estimateur de l'ensemble V, noté $\hat{V}$, pour chacun des G points du maillage ;
  - calculer une fonction-coût, notée $\varphi(S)$, représentative de la différence ou de la vraisemblance entre $\hat{V}$ et les valeurs modélisées de l'ensemble V extraites aux G points du maillage, notées $\overline{V}$;
  - extraire la distribution spatiale $S_{opt}$ qui minimise ou maximise la fonction-coût selon que la fonction-coût est représentative de la différence ou de la vraisemblance entre $\hat{V}$ et $\overline{V}$.

Ainsi, un tel système de mesure selon l'invention permet d'obtenir des mesures reflétant et rendant compte de la qualité de l'air dans la zone d'observation, autorisant une résolution spatiale supérieure à l'état de la technique, tout en s'affranchissant de mesures préalables locales. En effet, une telle cartographie et de tels moyens de calcul permettent, en conjugaison, l'extraction d'une distribution spatiale optimale des moyens de mesure à partir de valeurs modélisées.

### <u>Définitions</u>

- Par « cartographie », on entend une représentation spatiale des valeurs modélisées, pouvant être par exemple en deux dimensions (2D) ou en trois dimensions (3D).

- Par «représentatives de la grandeur physique », on entend que les valeurs modélisées représentent directement la grandeur physique, ou représentent indirectement la grandeur physique par corrélation.
- N est un nombre entier naturel.
- Les « N positions » des moyens de mesure sont définies par des coordonnées spatiales, par exemple des coordonnées cartésiennes (x, y, z).
- Les « N trajectoires » des moyens de mesure sont N ensembles de T positions successives au cours du temps. Les N trajectoires sont définies par des coordonnées spatiales, par exemple des coordonnées cartésiennes (x, y, z), et par une composante temporelle ($t_i$), i étant compris entre 1 et T.
- Par « distribution spatiale », on entend la répartition des N positions (ou des N trajectoires) dans l'espace de la zone d'observation. On peut parler de distribution volumique ou de densité volumique dans le cas 3D.
- Par « maillage », on entend la discrétisation spatiale de la zone d'observation.
- Par « estimateur », on entend un estimateur statistique où les N positions (ou les N trajectoires) sont des variables aléatoires.
- Par « fonction-coût », on entend toute fonction capable de quantifier la cohérence entre $\hat{V}$ et $\overline{V}$. On parle également de fonction-objectif.

Selon l'invention, les moyens de calcul sont configurés pour calculer l'estimateur de l'ensemble V selon la formule :

$$\widehat{V}_j = \frac{\sum_{i=1}^{N} V_i\, w_{ij}}{\sum_{i=1}^{N} w_{ij}}, \text{j} \in [\![1, G]\!]$$

où :

- $V_i$ sont les valeurs modélisées de l'ensemble V extraites pour les positions ou pour les trajectoires des moyens de mesure présentant la distribution spatiale donnée S,
- $w_{ij}$ est une fonction de transfert entre $s_i$ et $m_j$, où $s_i$ sont les positions ou les trajectoires des moyens de mesure présentant la distribution spatiale donnée S, et où $m_j$ sont les positions des points du maillage dans la zone d'observation.

Ainsi, un avantage procuré par la fonction de transfert est de pondérer les valeurs $V_i$ en tenant compte des positions relatives $s_i$ et $m_j$, par exemple selon une distance ou un angle issu des positions relatives s; et m

Le système de mesure selon l'invention peut comporter une ou plusieurs des caractéristiques suivantes.

Selon une caractéristique de l'invention, les moyens de calcul sont configurés pour calculer l'estimateur de l'ensemble V selon la formule :

$$\widehat{V}_j = \frac{\sum_{i=1}^{N} V_i\, \big[d(m_j, s_i)\big]^{-2}}{\sum_{i=1}^{N} \big[d(m_j, s_i)\big]^{-2}}, \text{j} \in [\![1, G]\!]$$

où :

- $m_j$ sont les positions des points du maillage dans la zone d'observation,
- $s_i$ sont les positions ou les trajectoires des moyens de mesure présentant la distribution spatiale donnée S,
- $V_i$ sont les valeurs modélisées de l'ensemble V extraites pour les positions ou pour les trajectoires $s_i$,
- d est une distance entre une position d'un point du maillage et une position ou une trajectoire des moyens de mesure dans la zone d'observation.

Ainsi, un avantage procuré par un tel estimateur est sa simplicité de mise en oeuvre pour les calculs.

Selon une caractéristique de l'invention, la fonction-coût est représentative de la différence entre $\hat{V}$ et $\overline{V}$, et la fonction-coût est une norme de la différence entre $\hat{V}$ et $\overline{V}$.

Ainsi, la minimisation d'une telle fonction-coût permet d'envisager l'obtention d'une distribution spatiale $S_{opt}$ reflétant au mieux la qualité de l'air dans la zone d'observation en référence aux valeurs modélisées.

Selon une caractéristique de l'invention, la fonction-coût est représentative de la différence entre $\hat{V}$ et $\overline{V}$, et les

moyens de calcul sont configurés pour calculer la fonction-coût selon la formule :

$$\varphi(S) = \frac{1}{G} \sum_{j=1}^{G} \left( \widehat{V_j} - \overline{V_j} \right)^2$$

où $\overline{V_j}$ sont les valeurs modélisées de l'ensemble V extraites pour les points du maillage.

Selon une caractéristique de l'invention, la fonction-coût est représentative de la différence entre $\widehat{V}$ et $\overline{V}$, et dans lequel les moyens de calcul sont configurés pour effectuer une modification aléatoire de la distribution spatiale donnée S selon une probabilité, notée $p_{ji}$, vérifiant :

$$p_{ji} = 1 \; si \; \varphi\left(S^{(j)}\right) \le \varphi\left(S^{(i)}\right)$$

$$p_{ji} = exp\left( \frac{\varphi(S^{(i)}) - \varphi(S^{(j)})}{c} \right) \; si \; \varphi\left(S^{(j)}\right) > \varphi\left(S^{(i)}\right)$$

où :

- $S^{(i)}$ est une distribution spatiale donnée initiale,
- $S^{(j)}$ est une distribution spatiale donnée modifiée aléatoirement,
- c est un paramètre ;

et les moyens de calcul sont configurés pour itérer la modification aléatoire de la distribution spatiale donnée S jusqu'à l'extraction de $S_{opt}$.

Ainsi, un avantage procuré est d'obtenir un algorithme robuste vis-à-vis du choix de la distribution spatiale donnée initiale, c'est-à-dire que la distribution spatiale $S_{opt}$ ne dépend pas de la distribution spatiale donnée initiale.

Selon une caractéristique de l'invention, la fonction-coût est représentative de la vraisemblance entre $\widehat{V}$ et $\overline{V}$, et la fonction-coût comprend un produit scalaire entre $\widehat{V}$ et $\overline{V}$, de préférence normalisé.

Ainsi, la maximisation d'une telle fonction-coût permet d'envisager l'obtention d'une distribution spatiale $S_{opt}$ reflétant au mieux la qualité de l'air dans la zone d'observation en référence aux valeurs modélisées.

Selon une caractéristique de l'invention, la fonction-coût est représentative de la vraisemblance entre $\widehat{V}$ et $\overline{V}$, et dans lequel les moyens de calcul sont configurés pour effectuer une modification aléatoire de la distribution spatiale donnée S selon une probabilité, notée $p_{ji}$, vérifiant :

$$p_{ji} = 1 \; si \; \varphi\left(S^{(j)}\right) \ge \varphi\left(S^{(i)}\right)$$

$$p_{ji} = exp\left( \frac{\varphi(S^{(j)}) - \varphi(S^{(i)})}{c} \right) \; si \; \varphi\left(S^{(j)}\right) < \varphi\left(S^{(i)}\right)$$

où :

- $S^{(i)}$ est une distribution spatiale donnée initiale,
- $S^{(j)}$ est une distribution spatiale donnée modifiée aléatoirement,
- c est un paramètre ;

et les moyens de calcul sont configurés pour itérer la modification aléatoire de la distribution spatiale donnée S jusqu'à l'extraction de $S_{opt}$.

Ainsi, un avantage procuré est d'obtenir un algorithme robuste vis-à-vis du choix de la distribution spatiale donnée initiale, c'est-à-dire que la distribution spatiale $S_{opt}$ ne dépend pas de la distribution spatiale donnée initiale.

Selon une caractéristique de l'invention, les moyens de mesure sont agencés dans la zone d'observation de manière à posséder N positions ou N trajectoires présentant la distribution spatiale $S_{opt}$.

Selon une caractéristique de l'invention, le système de mesure comporte des moyens d'activation configurés pour activer les moyens de mesure possédant N positions ou N trajectoires présentant la distribution spatiale la plus proche de $S_{opt}$.

Ainsi, un avantage procuré est d'optimiser la consommation électrique des moyens de mesure lorsque ceux-ci comportent un nombre total $N_{tot}$ de positions ou de trajectoires dans la zone d'observation vérifiant $N_{tot} > N$. En effet, il est alors possible de désactiver les moyens de mesure dont les positions ou les trajectoires dans la zone d'observation n'appartiennent pas aux N positions ou N trajectoires présentant la distribution spatiale la plus proche de $S_{opt}$.

Selon une caractéristique de l'invention, la zone d'observation comporte un nombre M de positions autorisées ou un nombre M de trajectoires autorisées, vérifiant M>N, dans lesquelles les moyens de mesure sont respectivement autorisés à posséder des positions ou des trajectoires.

Selon une caractéristique de l'invention, les moyens de mesure comportent au moins N dispositifs de mesure équipant chacun un véhicule, le véhicule étant de préférence routier ou aérien.

Ainsi, un avantage procuré est de pouvoir utiliser la circulation de véhicules dans la zone d'observation comme déploiement de dispositifs de mesure selon différentes distributions spatiales de positions ou de trajectoires.

Selon une caractéristique de l'invention, les moyens de mesure comportent au moins N dispositifs de mesure équipant chacun un mobilier urbain, le mobilier urbain étant de préférence sélectionné dans le groupe comportant des feux de circulation et des abribus.

Ainsi, un avantage procuré est d'éviter de créer des emplacements dédiés pour les dispositifs de mesure.

Selon une caractéristique de l'invention, les moyens de mesure comportent des capteurs spectroscopiques, de préférence des capteurs infrarouges non-dispersifs.

Ainsi, un avantage procuré est leur compacité par rapport à des stations de mesure conventionnelles, permettant une densité spatiale plus élevée et à moindre coût.

L'invention a également pour objet un procédé de mesure d'au moins une grandeur physique représentative de la qualité de l'air dans une zone d'observation, le procédé comportant les étapes :

a) prévoir une cartographie de la zone d'observation, comprenant un ensemble, noté V, de valeurs modélisées représentatives de la grandeur physique ;

b) prévoir des moyens de mesure de la grandeur physique, possédant un nombre N de positions ou un nombre N de trajectoires dans la zone d'observation, les N positions ou les N trajectoires étant destinées à présenter une distribution spatiale, notée $S_{opt}$ ;

c) effectuer un maillage de la zone d'observation, le maillage comprenant un nombre G de points ;

d) calculer, pour une distribution spatiale donnée des N positions ou des N trajectoires, notée S, un estimateur de l'ensemble V, noté $\hat{V}$, pour chacun des G points du maillage ;

e) calculer une fonction-coût, notée $\varphi(S)$, représentative de la différence ou de la vraisemblance entre $\hat{V}$ et les valeurs modélisées de l'ensemble V extraites aux G points du maillage, notées $\overline{V}$ ;

f) extraire la distribution spatiale $S_{opt}$ qui minimise ou maximise la fonction-coût selon que la fonction-coût est représentative de la différence ou de la vraisemblance entre $\hat{V}$ et $\overline{V}$ ;

g) agencer les moyens de mesure dans la zone d'observation de sorte que les N positions ou les N trajectoires présentent la distribution spatiale $S_{opt}$.

## Brève description des dessins

D'autres caractéristiques et avantages apparaîtront dans l'exposé détaillé de différents modes de réalisation de l'invention, l'exposé étant assorti d'exemples et de référence aux dessins joints.

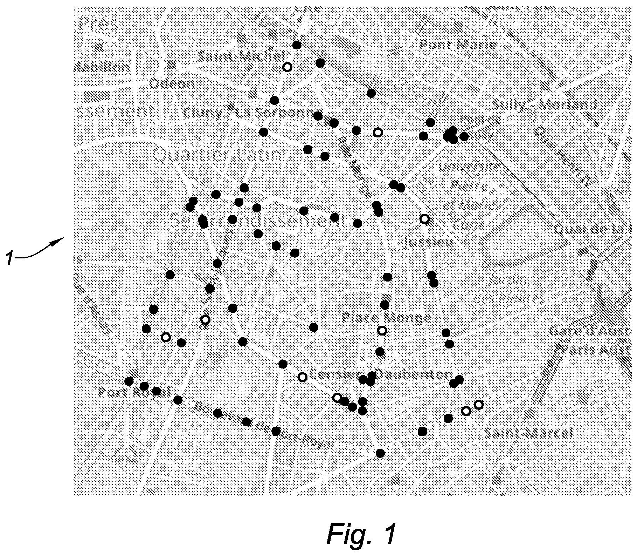

Figure 1 est une vue schématique partielle d'un système de mesure selon l'invention, illustrant des moyens de mesure, agencés dans une zone d'observation (un quartier de la ville de Paris), et possédant N positions présentant la distribution spatiale $S_{opt}$ (ronds blancs, N=10), sélectionnées parmi M positions autorisées (ronds noirs).

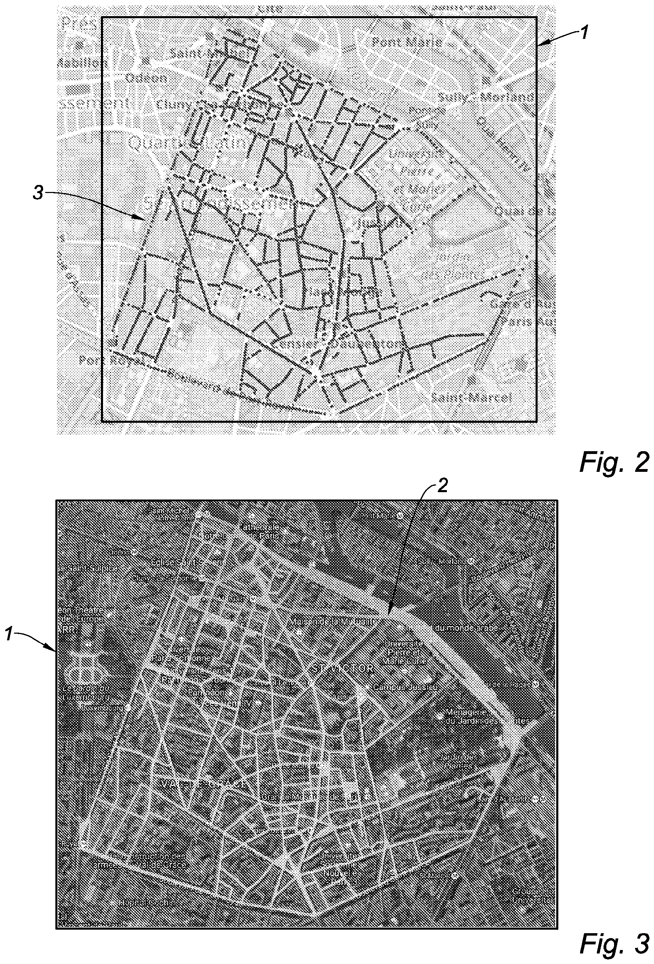

Figure 2 est une vue schématique partielle d'un système de mesure selon l'invention, illustrant un maillage d'une zone d'observation (un quartier de la ville de Paris).

Figure 3 est une vue schématique partielle d'un système de mesure selon l'invention, illustrant une cartographie d'une zone d'observation (un quartier de la ville de Paris), la cartographie comprenant un ensemble de valeurs modélisées représentatives de la grandeur physique à mesurer.

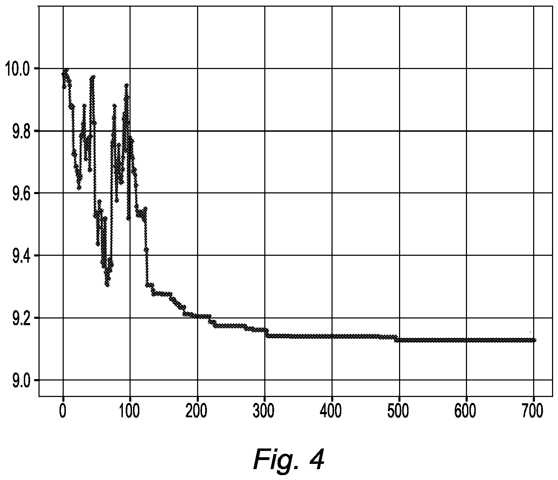

Figure 4 est un graphique illustrant en ordonnées la fonction-coût et en abscisses le nombre d'itérations (i.e. le nombre de modifications de la distribution spatiale des N positions ou N trajectoires).

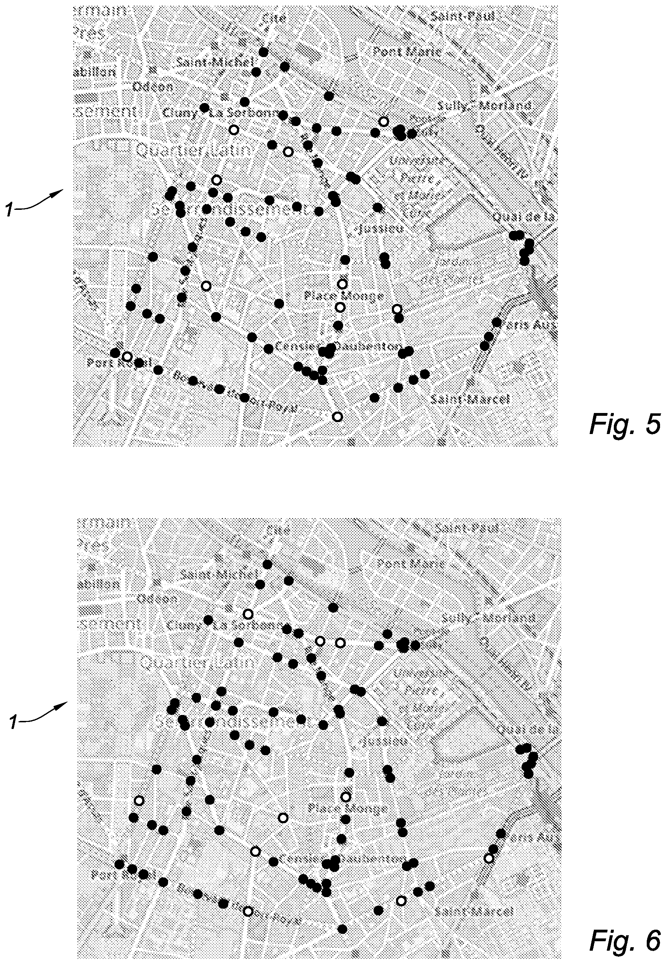

Figures 5 à 7 sont des vues analogues à la figure 1, illustrant respectivement une distribution spatiale initiale (ronds blancs) des N positions (N=10), une distribution spatiale intermédiaire (ronds blancs) des N positions, et la distribution spatiale $S_{opt}$ (ronds blancs) des N positions, sélectionnées parmi M positions autorisées (ronds noirs).

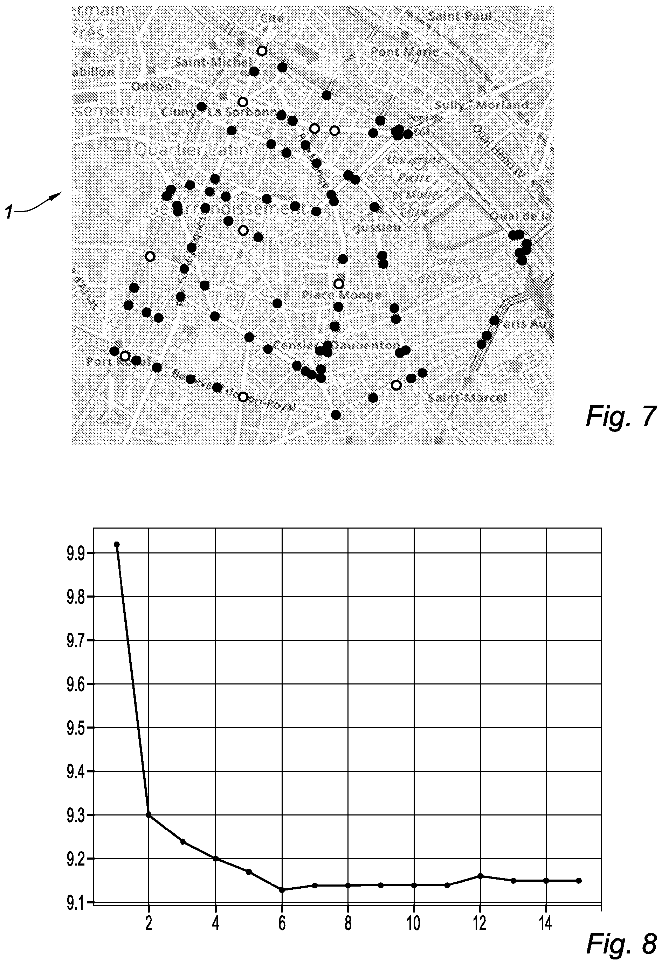

Figure 8 est un graphique illustrant en ordonnées la fonction-coût et en abscisses le nombre N de positions ou de trajectoires.

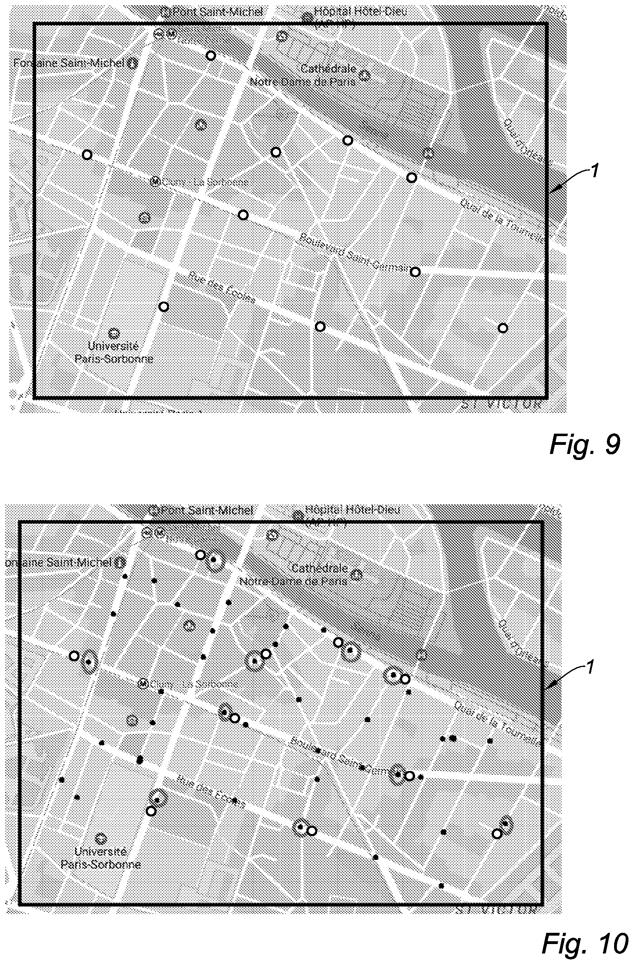

Figure 9 est une vue schématique partielle d'un système de mesure selon l'invention, illustrant des moyens de mesure, agencés dans une zone d'observation (un quartier de la ville de Paris), et possédant N positions présentant la distribution spatiale $S_{opt}$ (ronds blancs, N=10).

Figure 10 est une vue schématique partielle d'un système de mesure selon l'invention, comportant des moyens d'activation configurés pour activer des moyens de mesure fixes dans la zone d'observation, et possédant N positions présentant la distribution spatiale la plus proche de $S_{opt}$ (ronds noirs encerclés, N=10). La distribution spatiale $S_{opt}$ extraite est illustrée par des ronds blancs. L'ensemble des positions fixes des moyens de mesure est illustré par des ronds noirs.

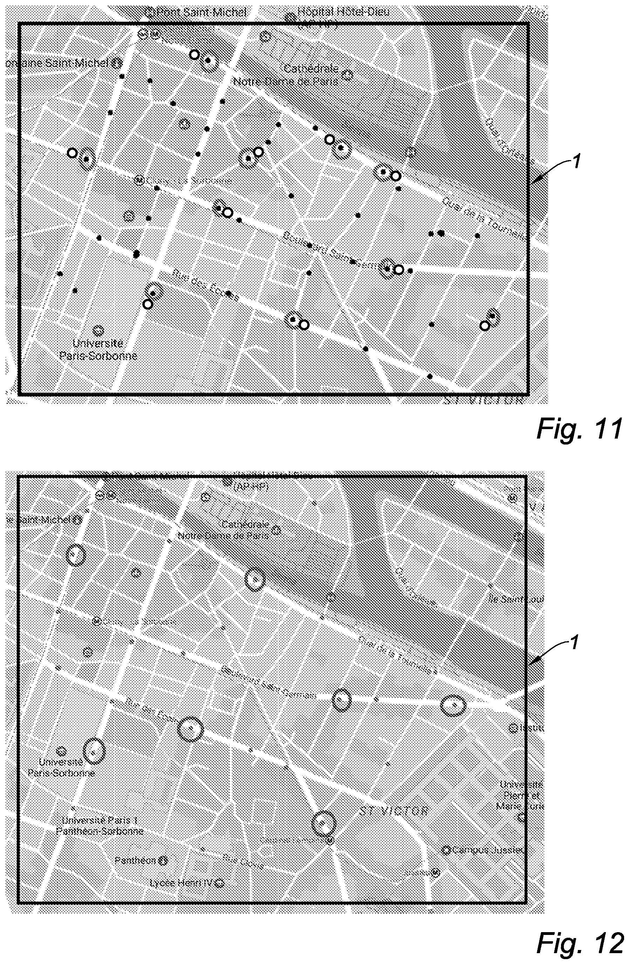

Figure 11 est une vue schématique partielle d'un système de mesure selon l'invention, comportant des moyens d'activation configurés pour activer des moyens de mesure mobiles dans la zone d'observation, et possédant N positions, à un instant donné, présentant la distribution spatiale la plus proche de $S_{opt}$ (ronds noirs encerclés, N=10). La distribution spatiale $S_{opt}$ extraite est illustrée par des ronds blancs. L'ensemble des positions des moyens de mesure, à un instant donné, est illustré par des ronds noirs.

Figure 12 est une vue schématique partielle d'un système de mesure selon l'invention, illustrant des moyens de mesure, agencés dans une zone d'observation, et possédant N positions présentant la distribution spatiale $S_{opt}$ (points encerclés, N=7), sélectionnées parmi M positions autorisées (points).

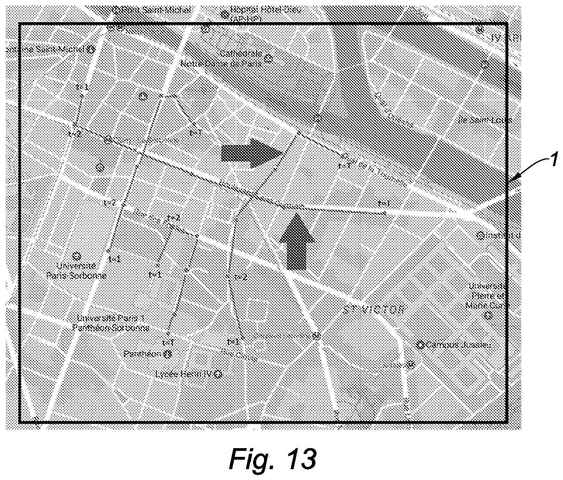

Figure 13 est une vue schématique partielle d'un système de mesure selon l'invention, illustrant des moyens de mesure, agencés dans une zone d'observation, et possédant N trajectoires présentant la distribution spatiale $S_{opt}$ (pointées par les flèches, N=2), sélectionnées parmi M trajectoires autorisées (M=4).

## Exposé détaillé des modes de réalisation

Les éléments identiques ou assurant la même fonction porteront les mêmes références pour les différents modes de réalisation, par souci de simplification.

Un objet de l'invention est un système de mesure d'au moins une grandeur physique représentative de la qualité de l'air dans une zone d'observation selon la revendication 1.

## Grandeur physique

A titre d'exemples non limitatifs, la ou les grandeurs physiques peuvent être des concentrations de molécules polluantes ou des concentrations de particules nocives pour l'environnement. On peut citer le monoxyde de carbone CO, le dioxyde de soufre SOz, le dioxyde d'azote NOz, les matières particulaires en suspension (SPM pour « *Suspended Particulate Matter*»), les matières particulaires en suspension respirables (RSPM pour «*Respirable Suspended Particulate Matter*»), les hydrocarbures aromatiques polycycliques (PAHs pour «*Polycyclic Aromatic Hydrocarbons* »), l'ozone $O_3$.

## Zone d'observation

A titre d'exemples non limitatifs, la zone d'observation 1 peut être une zone urbaine, une zone périurbaine, une zone industrielle.

La zone d'observation 1 peut comporter un nombre M de positions autorisées ou un nombre M de trajectoires autorisées, vérifiant M>N, dans lesquelles les moyens de mesure sont respectivement autorisés à posséder des positions ou des trajectoires. M est un entier naturel.

## Cartographie

Il existe dans l'état de la technique des cartographies 2 de zones d'observation 1, généralement urbaines, pouvant comprendre des valeurs modélisées de la distribution spatiale de concentrations en molécules polluantes ou en particules nocives. Ces cartographies 2 sont classiquement issues de modèles développés pour étudier la pollution atmosphérique et son évolution temporelle. A titre d'exemple non limitatif, la publication de R. Berkowicz et al., « Modeling trafic pollution in streets », National Environmental Research Institute, datée de janvier 1997, décrit un modèle de dispersion spatiale de polluants adapté aux spécificités des zones urbaines. En milieu urbain, la topographie particulière formée de rues séparées par des bâtiments justifie une approche spécifique, tenant compte de la formation de vortex de circulation d'air dans les rues, ces vortex jouant un rôle déterminant dans la dispersion de la pollution atmosphérique. De tels modèles sont désignés par les termes « *Street Canyon Model* » ou « *Street Model* ». La publication précitée décrit un modèle d'estimation de la pollution en milieu urbain désigné par l'acronyme OSPM (« *Operational Street*

*Pollution Model* »), c'est-à-dire un modèle opérationnel de pollution urbaine. Selon ce modèle, à partir de l'émission d'un polluant dans une rue, dépendant du nombre de véhicules et d'une émission moyenne par véhicule, le modèle prend en compte le vortex de recirculation formé dans la rue, la turbulence aérologique résultant du trafic routier, la pollution ambiante, provenant d'autres rues, ainsi que le vent circulant dans la canopée, au-dessus du milieu urbain. La publication de R. Berkowicz « OSPM - A Parameterised Street Pollution Model », Environmental Monitoring and Assessment, 65(1), pp. 323-331, 2000, présente également les hypothèses sur lesquelles est fondé le modèle OSPM, ainsi qu'une validation expérimentale de ce modèle.

La cartographie 2 de l'ensemble V des valeurs modélisées peut représenter directement la grandeur physique mesurée. Selon une variante, la cartographie 2 de l'ensemble V des valeurs modélisées peut représenter indirectement, par corrélation, la grandeur physique mesurée. A titre d'exemples non limitatifs, on peut citer des valeurs modélisant les nuisances sonores, l'ensoleillement, le vent, la température, le trafic routier, les ondes électromagnétiques (radio, téléphone portable).

La cartographie 2 présente une résolution spatiale supérieure à celle du maillage 3.

**Moyens de mesure de la grandeur physique**

Les moyens de mesure comportent des dispositifs de mesure. Les dispositifs de mesure comportent avantageusement des capteurs spectroscopiques, de préférence des capteurs infrarouges non-dispersifs.

Selon un mode de réalisation, les moyens de mesure sont agencés dans la zone d'observation 1 de manière à posséder N positions ou N trajectoires présentant la distribution spatiale $S_{opt}$. Selon une variante, le système de mesure comporte des moyens d'activation configurés pour activer les moyens de mesure possédant N positions ou N trajectoires présentant la distribution spatiale la plus proche de $S_{opt}$. A titre d'exemple, les moyens d'activation peuvent être commandés depuis un poste central de régulation.

Selon un mode de réalisation, les moyens de mesure comportent au moins N dispositifs de mesure équipant chacun un véhicule, le véhicule étant de préférence routier ou aérien. A titre d'exemples non limitatifs, le véhicule routier peut être une voiture ou un bus ; le véhicule aérien peut un être un aéronef sans pilote tel qu'un drone. Selon une variante, les moyens de mesure comportent au moins N dispositifs de mesure équipant chacun un mobilier urbain, le mobilier urbain étant de préférence sélectionné dans le groupe comportant des feux de circulation et des abribus.

Exemple n°1

Dans cet exemple illustré à la figure 9, les moyens de mesure comportent N dispositifs de mesure fixes dans la zone d'observation 1. Les N dispositifs de mesure sont agencés dans la zone d'observation 1 de manière à posséder N positions présentant la distribution spatiale $S_{opt}$ (ronds blancs).

Exemple n°2

Dans cet exemple illustré à la figure 10, les moyens de mesure comportent un nombre total $N_{tot}$ de dispositifs de mesure ($N_{tot} > N$, ronds noirs) fixes dans la zone d'observation 1. Le système de mesure comporte des moyens d'activation configurés pour activer, sur une période donnée, les dispositifs de mesure possédant N positions présentant la distribution spatiale la plus proche de $S_{opt}$ (ronds noirs encerclés). La distribution spatiale $S_{opt}$ extraite est illustrée par des ronds blancs.

Exemple n°3

Dans cet exemple illustré à la figure 11, les moyens de mesure comportent un nombre total $N_{tot}$ de dispositifs de mesure ($N_{tot} > N$, ronds noirs) mobiles dans la zone d'observation 1. Le système de mesure comporte des moyens d'activation configurés pour activer, à instant donné, les dispositifs de mesure possédant N positions présentant la distribution spatiale la plus proche de $S_{opt}$ (ronds noirs encerclés). La distribution spatiale $S_{opt}$ extraite est illustrée par des ronds blancs.

Exemple n°4

Dans cet exemple illustré à la figure 12, la zone d'observation 1 comporte M positions autorisées (points). Les moyens de mesure comportent N dispositifs de mesure agencés dans la zone d'observation 1 de manière à posséder N positions (parmi les M positions autorisées) présentant la distribution spatiale $S_{opt}$ (points encerclés).

Exemple n°5

Dans cet exemple illustré à la figure 13, la zone d'observation 1 comporte M trajectoires autorisées. Les moyens de mesure comportent N dispositifs de mesure agencés dans la zone d'observation 1 de manière à posséder N trajectoires (parmi les M trajectoires autorisées) présentant la distribution spatiale $S_{opt}$ (pointées par les flèches).

**Moyens de calcul de la distribution spatiale $S_{opt}$**

Les moyens de calcul sont avantageusement mis en oeuvre par ordinateur ou par tout dispositif programmable comportant les instructions de calcul adaptées.

**Estimateur de l'ensemble V**

Les moyens de calcul sont configurés pour calculer l'estimateur de l'ensemble V selon la formule :

$$\widehat{V}_j = \frac{\sum_{i=1}^{N} V_i\, w_{ij}}{\sum_{i=1}^{N} w_{ij}}, j \in [\![1, G]\!]$$

où :

- $V_i$ sont les valeurs modélisées de l'ensemble V extraites pour les positions ou pour les trajectoires des moyens de mesure présentant la distribution spatiale donnée S,
- $w_{ij}$ est une fonction de transfert entre $s_i$ et $m_j$, où $s_i$ sont les positions ou les trajectoires des moyens de mesure présentant la distribution spatiale donnée S, et où $m_j$ sont les positions des points du maillage 3 dans la zone d'observation 1.

La fonction de transfert permet de pondérer les valeurs $V_i$ en tenant compte des positions relatives $s_i$ et $m_j$, par exemple selon une distance ou un angle issu des positions relatives $s_i$ et $m_j$.
Les moyens de calcul sont avantageusement configurés pour calculer l'estimateur de l'ensemble V selon la formule :

$$\widehat{V}_j = \frac{\sum_{i=1}^{N} V_i\, [d(m_j, s_i)]^{-2}}{\sum_{i=1}^{N} [d(m_j, s_i)]^{-2}}, j \in [\![1, G]\!]$$

où :

- $m_j$ sont les positions des points du maillage 3 dans la zone d'observation 1,
- $s_i$ sont les positions ou les trajectoires des moyens de mesure présentant la distribution spatiale donnée S,
- $V_i$ sont les valeurs modélisées de l'ensemble V extraites pour les positions ou pour les trajectoires $s_i$,
- $d$ est une distance entre une position d'un point du maillage 3 et une position ou une trajectoire des moyens de mesure dans la zone d'observation 1.

La distance d peut être une distance routière ou une distance euclidienne (i.e. à vol d'oiseau).

**Fonction-coût**

Lorsque la fonction-coût est représentative de la différence entre $\hat{V}$ et $\overline{V}$, la fonction-coût est avantageusement une norme de la différence entre $\hat{V}$ et $\overline{V}$. Lorsque la fonction-coût est représentative de la différence entre $\hat{V}$ et $\overline{V}$, les moyens de calcul sont avantageusement configurés pour calculer la fonction-coût selon la formule :

$$\varphi(S) = \frac{1}{G} \sum_{j=1}^{G} \left(\widehat{V}_j - \bar{V}_j\right)^2$$

où $\overline{V}_j$ sont les valeurs modélisées de l'ensemble V extraites pour les points du maillage 3.

Lorsque la fonction-coût est représentative de la vraisemblance entre $\hat{V}$ et $\overline{V}$, la fonction-coût comprend avantageusement un produit scalaire entre $\hat{V}$ et $\overline{V}$, de préférence normalisé.

**Algorithme d'extraction de S$_{opt}$**

Lorsque la fonction-coût est représentative de la différence entre $\hat{V}$ et $\overline{V}$, les moyens de calcul sont avantageusement configurés pour effectuer une modification aléatoire de la distribution spatiale donnée S selon une probabilité, notée p$_{ji}$, vérifiant :

$$p_{ji} = 1 \; si \; \varphi\left(S^{(j)}\right) \leq \varphi\left(S^{(i)}\right)$$

$$p_{ji} = exp\left(\frac{\varphi(S^{(i)}) - \varphi(S^{(j)})}{c}\right) \; si \; \varphi\left(S^{(j)}\right) > \varphi\left(S^{(i)}\right)$$

où :

- S$^{(i)}$ est une distribution spatiale donnée initiale,
- S$^{(j)}$ est une distribution spatiale donnée modifiée aléatoirement,
- c est un paramètre.

Si l'on considère une itération, notée t, le paramètre c est de préférence choisi de sorte que : $c(t+1) = \alpha c(t)$ ; $\alpha \approx 1$.

Les moyens de calcul sont configurés pour itérer la modification aléatoire de la distribution spatiale donnée S jusqu'à l'extraction de S$_{opt}$. Les moyens de calcul sont configurés pour appliquer un critère d'arrêt aux itérations. A titre d'exemple, le critère d'arrêt peut être défini lorsque la différence $|\varphi(S^{(j+1)}) - \varphi(S^{(j)})|$ est inférieure à un seuil prédéterminé.

Lorsque la fonction-coût est représentative de la vraisemblance entre $\hat{V}$ et $\overline{V}$, les moyens de calcul sont avantageusement configurés pour effectuer une modification aléatoire de la distribution spatiale donnée S selon une probabilité, notée p$_{ji}$, vérifiant :

$$p_{ji} = 1 \; si \; \varphi\left(S^{(j)}\right) \geq \varphi\left(S^{(i)}\right)$$

$$p_{ji} = exp\left(\frac{\varphi(S^{(j)}) - \varphi(S^{(i)})}{c}\right) \; si \; \varphi\left(S^{(j)}\right) < \varphi\left(S^{(i)}\right)$$

où :

- S$^{(i)}$ est une distribution spatiale donnée initiale,
- S$^{(j)}$ est une distribution spatiale donnée modifiée aléatoirement,
- c est un paramètre.

Si l'on considère une itération, notée t, le paramètre c est de préférence choisi de sorte que : $c(t+1) = \alpha c(t)$ ; $\alpha \approx 1$.

Les moyens de calcul sont configurés pour itérer la modification aléatoire de la distribution spatiale donnée S jusqu'à l'extraction de S$_{opt}$. Les moyens de calcul sont configurés pour appliquer un critère d'arrêt aux itérations. A titre d'exemple, le critère d'arrêt peut être défini lorsque la différence $|\varphi(S^{(j+1)}) - \varphi(S^{(j)})|$ est inférieure à un seuil prédéterminé.

Comme illustré à la figure 4, un tel algorithme converge à partir d'un certain nombre d'itérations. Comme illustré à la figure 8, il est également possible de faire varier le nombre N de positions ou de trajectoires afin de déterminer la distribution spatiale S$_{opt}$ avec le nombre N minimum (N=6 à la figure 6) permettant de minimiser ou maximiser la fonction-coût selon que la fonction-coût est représentative de la différence ou de la vraisemblance entre $\hat{V}$ et $\overline{V}$.

L'algorithme fonctionne de manière analogue avec N positions ou N trajectoires des moyens de mesure. Dans le cas où les moyens de mesure possèdent N trajectoires dans la zone d'observation 1, les positions successives des N trajectoires sont échantillonnées à T instants. V et $\overline{V}$ sont alors des vecteurs comportant T éléments. Dans le cas où les moyens de mesure possèdent N positions dans la zone d'observation 1, $\hat{V}$ et $\overline{V}$ sont des scalaires.

L'invention ne se limite pas aux modes de réalisation exposés. L'homme du métier est mis à même de considérer leurs combinaisons techniquement opérantes faisant parties du champ de protection établi par le jeu de revendications en annexe.

**Revendications**

1. Système de mesure d'au moins une grandeur physique représentative de la qualité de l'air dans une zone d'observation (1), le système comportant :

   - une cartographie (2) de la zone d'observation (1), comprenant un ensemble, noté V, de valeurs modélisées représentatives de la grandeur physique ;
   - des moyens de mesure de la grandeur physique, possédant un nombre N de positions ou un nombre N de trajectoires dans la zone d'observation (1), les N positions ou les N trajectoires étant destinées à présenter une distribution spatiale, notée $S_{opt}$, dans la zone d'observation (1) ;
   - des moyens de calcul de la distribution spatiale $S_{opt}$, configurés pour :

      • effectuer un maillage (3) de la zone d'observation (1), le maillage (3) comprenant un nombre G de points ;
      • calculer, pour une distribution spatiale donnée des N positions ou des N trajectoires, notée S, un estimateur de l'ensemble V, noté $\hat{V}$, pour chacun des G points du maillage (3) selon la formule :

$$\widehat{V}_j = \frac{\sum_{i=1}^{N} V_i\, w_{ij}}{\sum_{i=1}^{N} w_{ij}}, j \in [\![1, G]\!]$$

où :

   - $V_i$ sont les valeurs modélisées de l'ensemble V extraites pour les positions ou pour les trajectoires des moyens de mesure présentant la distribution spatiale donnée S,
   - $w_{ij}$ est une fonction de transfert entre $s_i$ et $m_j$, où $s_i$ sont les positions ou les trajectoires des moyens de mesure présentant la distribution spatiale donnée S, et où $m_j$ sont les positions des points du maillage (3) dans la zone d'observation (1) ;

      • calculer une fonction-coût, notée $\varphi(S)$, représentative de la différence ou de la vraisemblance entre $\hat{V}$ et les valeurs modélisées de l'ensemble V extraites aux G points du maillage, notées $\overline{V}$;
      • extraire la distribution spatiale $S_{opt}$ qui minimise ou maximise la fonction-coût selon que la fonction-coût est représentative de la différence ou de la vraisemblance entre $\hat{V}$ et $\overline{V}$.

2. Système selon la revendication 1, dans lequel les moyens de calcul sont configurés pour calculer l'estimateur de l'ensemble V selon la formule :

$$\widehat{V}_j = \frac{\sum_{i=1}^{N} V_i\, \big[d(m_j, s_i)\big]^{-2}}{\sum_{i=1}^{N} \big[d(m_j, s_i)\big]^{-2}}, j \in [\![1, G]\!]$$

où :

   - $m_j$ sont les positions des points du maillage (3) dans la zone d'observation (1),
   - $s_i$ sont les positions ou les trajectoires des moyens de mesure présentant la distribution spatiale donnée S,
   - $V_i$ sont les valeurs modélisées de l'ensemble V extraites pour les positions ou pour les trajectoires $s_i$,
   - d est une distance entre une position d'un point du maillage (3) et une position ou une trajectoire des moyens de mesure dans la zone d'observation (1).

3. Système selon la revendication 1 ou 2, dans lequel la fonction-coût est représentative de la différence entre $\hat{V}$ et $\overline{V}$, et la fonction-coût est une norme de la différence entre $\hat{V}$ et $\overline{V}$.

4. Système selon l'une des revendications 1 à 3, dans lequel la fonction-coût est représentative de la différence entre $\hat{V}$ et $\overline{V}$, et dans lequel les moyens de calcul sont configurés pour calculer la fonction-coût selon la formule :

$$\varphi(S) = \frac{1}{G} \sum_{j=1}^{G} \left( \widehat{V}_j - \overline{V}_j \right)^2$$

où $\overline{V}_j$ sont les valeurs modélisées de l'ensemble V extraites pour les points du maillage (3).

5. Système selon l'une des revendications 1 à 4, dans lequel la fonction-coût est représentative de la différence entre $\hat{V}$ et $\overline{V}$, et dans lequel les moyens de calcul sont configurés pour effectuer une modification aléatoire de la distribution spatiale donnée S selon une probabilité, notée $p_{ji}$, vérifiant :

$$p_{ji} = 1 \; si \; \varphi\left(S^{(j)}\right) \leq \varphi\left(S^{(i)}\right)$$

$$p_{ji} = exp \left( \frac{\varphi(S^{(i)}) - \varphi(S^{(j)})}{c} \right) \; si \; \varphi\left(S^{(j)}\right) > \varphi\left(S^{(i)}\right)$$

où :

- $S^{(i)}$ est une distribution spatiale donnée initiale,
- $S^{(j)}$ est une distribution spatiale donnée modifiée aléatoirement,
- c est un paramètre ;

et dans lequel les moyens de calcul sont configurés pour itérer la modification aléatoire de la distribution spatiale donnée S jusqu'à l'extraction de $S_{opt}$.

6. Système selon la revendication 1 ou 2, dans lequel la fonction-coût est représentative de la vraisemblance entre $\hat{V}$ et $\overline{V}$, et la fonction-coût comprend un produit scalaire entre $\hat{V}$ et $\overline{V}$, de préférence normalisé.

7. Système selon la revendication 1 ou 2 ou selon la revendication 6, dans lequel la fonction-coût est représentative de la vraisemblance entre $\hat{V}$ et $\overline{V}$, et dans lequel les moyens de calcul sont configurés pour effectuer une modification aléatoire de la distribution spatiale donnée S selon une probabilité, notée $p_{ji}$, vérifiant :

$$p_{ji} = 1 \; si \; \varphi\left(S^{(j)}\right) \geq \varphi\left(S^{(i)}\right)$$

$$p_{ji} = exp \left( \frac{\varphi(S^{(j)}) - \varphi(S^{(i)})}{c} \right) \; si \; \varphi\left(S^{(j)}\right) < \varphi\left(S^{(i)}\right)$$

où :

- $S^{(i)}$ est une distribution spatiale donnée initiale,
- $S^{(j)}$ est une distribution spatiale donnée modifiée aléatoirement,
- c est un paramètre ;

et dans lequel les moyens de calcul sont configurés pour itérer la modification aléatoire de la distribution spatiale donnée S jusqu'à l'extraction de $S_{opt}$.

8. Système selon l'une des revendications 1 à 7, dans lequel les moyens de mesure sont agencés dans la zone d'observation (1) de manière à posséder N positions ou N trajectoires présentant la distribution spatiale $S_{opt}$.

9. Système selon l'une des revendications 1 à 7, comportant des moyens d'activation configurés pour activer les moyens de mesure possédant N positions ou N trajectoires présentant la distribution spatiale la plus proche de $S_{opt}$.

10. Système selon l'une des revendications 1 à 9, dans lequel la zone d'observation (1) comporte un nombre M de positions autorisées ou un nombre M de trajectoires autorisées, vérifiant M>N, dans lesquelles les moyens de mesure sont respectivement autorisés à posséder des positions ou des trajectoires.

11. Système selon l'une des revendications 1 à 10, dans lequel les moyens de mesure comportent au moins N dispositifs de mesure équipant chacun un véhicule, le véhicule étant de préférence routier ou aérien.

12. Système selon l'une des revendications 1 à 10, dans lequel les moyens de mesure comportent au moins N dispositifs de mesure équipant chacun un mobilier urbain, le mobilier urbain étant de préférence sélectionné dans le groupe comportant des feux de circulation et des abribus.

13. Système selon l'une des revendications 1 à 12, dans lequel les moyens de mesure comportent des capteurs spectroscopiques, de préférence des capteurs infrarouges non-dispersifs.

14. Procédé de mesure d'au moins une grandeur physique représentative de la qualité de l'air dans une zone d'observation (1), le procédé comportant les étapes :

a) prévoir une cartographie (2) de la zone d'observation (1), comprenant un ensemble, noté V, de valeurs modélisées représentatives de la grandeur physique ;
b) prévoir des moyens de mesure de la grandeur physique, possédant un nombre N de positions ou un nombre N de trajectoires dans la zone d'observation (1), les N positions ou les N trajectoires étant destinées à présenter une distribution spatiale, notée $S_{opt}$ ;
c) effectuer un maillage (3) de la zone d'observation (1), le maillage (3) comprenant un nombre G de points ;
d) calculer, pour une distribution spatiale donnée des N positions ou des N trajectoires, notée S, un estimateur de l'ensemble V, noté $\widehat{V}$, pour chacun des G points du maillage (3) selon la formule :

$$\widehat{V}_{\mathrm{j}} = \frac{\sum_{i=1}^{N} \mathrm{V}_i \, w_{ij}}{\sum_{i=1}^{N} w_{ij}}, \mathrm{j} \in [\![1, G]\!]$$

où :

- $V_i$ sont les valeurs modélisées de l'ensemble V extraites pour les positions ou pour les trajectoires des moyens de mesure présentant la distribution spatiale donnée S,
- $w_{ij}$ est une fonction de transfert entre $s_i$ et $m_j$, où $s_i$ sont les positions ou les trajectoires des moyens de mesure présentant la distribution spatiale donnée S, et où $m_j$ sont les positions des points du maillage (3) dans la zone d'observation (1) ;

e) calculer une fonction-coût, notée $\varphi(S)$, représentative de la différence ou de la vraisemblance entre $\hat{V}$ et les valeurs modélisées de l'ensemble V extraites aux G points du maillage (3), notées $\overline{V}$ ;
f) extraire la distribution spatiale $S_{opt}$ qui minimise ou maximise la fonction-coût selon que la fonction-coût est représentative de la différence ou de la vraisemblance entre $\hat{V}$ et $\overline{V}$ ;
g) agencer les moyens de mesure dans la zone d'observation (1) de sorte que les N positions ou les N trajectoires présentent la distribution spatiale $S_{opt}$.

**Patentansprüche**

1. System zum Messen mindestens einer physikalischen Größe, die für die Qualität der Luft in einem Beobachtungsbereich (1) repräsentativ ist, wobei das System umfasst:

- eine Kartierung (2) des Beobachtungsbereichs (1), umfassend eine als V bezeichnete Menge von modellierten Werten, die für die physikalische Größe repräsentativ sind;

- Messmittel zum Messen der physikalischen Größe, die eine Anzahl N von Positionen oder eine Anzahl N von Trajektorien in dem Beobachtungsbereich (1) besitzen, wobei die N Positionen oder die N Trajektorien dazu bestimmt sind, eine als $S_{opt}$ bezeichnete räumliche Verteilung in dem Beobachtungsbereich (1) aufzuweisen;
- Rechenmittel zum Berechnen der räumlichen Verteilung $S_{opt}$, die dazu ausgestaltet sind:

    • ein Netz (3) des Beobachtungsbereichs (1) zu erstellen, wobei das Netz (3) eine Anzahl G von Punkten umfasst;
    • für eine als S bezeichnete gegebene räumliche Verteilung der N Positionen oder der N Trajektorien einen als $\hat{V}$ bezeichneten Schätzer der Menge V für jeden der G Punkte des Netzes (3) nach der folgenden Formel zu berechnen:

$$\hat{V}_j = \frac{\sum_{i=1}^N V_i\, w_{ij}}{\sum_{i=1}^N w_{ij}}, j \in [\![1, G]\!]$$

worin:

    - $V_i$ die modellierten Werte der Menge V sind, die für die Positionen oder für die Trajektorien der Messmittel extrahiert werden, die die gegebene räumliche Verteilung S aufweisen,
    - $w_{ij}$ eine Übertragungsfunktion zwischen $s_i$ und $m_j$ ist, wobei $s_i$ die Positionen oder die Trajektorien der Messmittel sind, die die gegebene räumliche Verteilung S aufweisen, und wobei $m_j$ die Positionen der Punkte des Netzes (3) in dem Beobachtungsbereich (1) sind;
    • eine als $\varphi(S)$ bezeichnete Kostenfunktion zu berechnen, die für die Differenz oder die Likelihood zwischen $\hat{V}$ und den als $\overline{V}$ bezeichneten modellierten Werten der Menge V repräsentativ ist, die an den G Punkten des Netzes extrahiert werden:
    • die räumliche Verteilung $S_{opt}$ zu extrahieren, die die Kostenfunktion minimiert oder maximiert, je nachdem, ob die Kostenfunktion für die Differenz oder die Likelihood zwischen $\hat{V}$ und $\overline{V}$ repräsentativ ist.

2. System nach Anspruch 1, wobei die Rechenmittel dazu ausgestaltet sind, den Schätzer der Menge V nach der folgenden Formel zu berechnen:

$$\hat{V}_j = \frac{\sum_{i=1}^N V_i\, [d(m_j, s_i)]^{-2}}{\sum_{i=1}^N [d(m_j, s_i)]^{-2}}, j \in [\![1, G]\!]$$

worin:

    - $m_j$ die Positionen der Punkte des Netzes (3) in dem Beobachtungsbereich (1) sind,
    - $s_i$ die Positionen oder die Trajektorien der Messmittel sind, die die gegebene räumliche Verteilung S aufweisen,
    - $V_i$ die modellierten Werte der Menge V sind, die für die Positionen oder für die Trajektorien $s_i$ extrahiert werden,
    - d ein Abstand zwischen einer Position eines Punkts des Netzes (3) und einer Position oder einer Trajektorie der Messmittel in dem Beobachtungsbereich (1) ist.

3. System nach Anspruch 1 oder 2, wobei die Kostenfunktion für die Differenz zwischen $\hat{V}$ und $\overline{V}$ repräsentativ ist und die Kostenfunktion eine Norm der Differenz zwischen $\hat{V}$ und $\overline{V}$ ist.

4. System nach einem der Ansprüche 1 bis 3, wobei die Kostenfunktion für die Differenz zwischen $\hat{V}$ und $\overline{V}$ repräsentativ ist und wobei die Rechenmittel dazu ausgestaltet sind, die Kostenfunktion nach der folgenden Formel zu berechnen:

$$\varphi(S) = \frac{1}{G} \sum_{j=1}^G (\hat{V}_j - \bar{V}_j)^2$$

worin $\overline{V}_j$ die modellierten Werte der Menge V sind, die für die Punkte des Netzes (3) extrahiert werden.

5. System nach einem der Ansprüche 1 bis 4, wobei die Kostenfunktion für die Differenz zwischen $\hat{V}$ und $\overline{V}$ repräsentativ ist und wobei die Rechenmittel dazu ausgestaltet sind, eine zufällige Änderung der gegebenen räumlichen Verteilung S gemäß einer als $p_{ji}$ bezeichneten Wahrscheinlichkeit vorzunehmen, für die gilt:

$$p_{ji} = 1 \; si \; \varphi(S^{(j)}) \leq \varphi(S^{(i)})$$

$$p_{ji} = exp\left(\frac{\varphi(S^{(i)}) - \varphi(S^{(j)})}{c}\right) wenn \; \varphi(S^{(j)}) > \varphi(S^{(i)})$$

worin:

- $S^{(i)}$ eine anfängliche gegebene räumliche Verteilung ist,
- $S^{(j)}$ eine zufällig geänderte gegebene räumliche Verteilung ist,
- c ein Parameter ist;

und wobei die Rechenmittel dazu ausgestaltet sind, die zufällige Änderung der gegebenen räumlichen Verteilung S bis zur Extraktion von $S_{opt}$ zu wiederholen.

6. System nach Anspruch 1 oder 2, wobei die Kostenfunktion für die Likelihood zwischen $\hat{V}$ und $\overline{V}$ repräsentativ ist und die Kostenfunktion ein Skalarprodukt zwischen $\hat{V}$ und $\overline{V}$ umfasst, das bevorzugt normiert ist.

7. System nach Anspruch 1 oder 2 oder nach Anspruch 6, wobei die Kostenfunktion für die Likelihood zwischen $\hat{V}$ und $\overline{V}$ repräsentativ ist und wobei die Rechenmittel dazu ausgestaltet sind, eine zufällige Änderung der gegebenen räumlichen Verteilung S gemäß einer als $p_{ji}$ bezeichneten Wahrscheinlichkeit vorzunehmen, für die gilt:

$$p_{ji} = 1 \; si \; \varphi(S^{(j)}) \geq \varphi(S^{(i)})$$

$$p_{ji} = exp\left(\frac{\varphi(S^{(j)}) - \varphi(S^{(i)})}{c}\right) wenn \; \varphi(S^{(j)}) < \varphi(S^{(i)})$$

worin:

- $S^{(i)}$ eine anfängliche gegebene räumliche Verteilung ist,
- $S^{(j)}$ eine zufällig geänderte gegebene räumliche Verteilung ist,
- c ein Parameter ist;

und wobei die Rechenmittel dazu ausgestaltet sind, die zufällige Änderung der gegebenen räumlichen Verteilung S bis zur Extraktion von $S_{opt}$ zu wiederholen.

8. System nach einem der Ansprüche 1 bis 7, wobei die Messmittel in dem Beobachtungsbereich (1) so angeordnet sind, dass sie N Positionen oder N Trajektorien besitzen, die die räumliche Verteilung $S_{opt}$ aufweisen.

9. System nach einem der Ansprüche 1 bis 7, das Aktivierungsmittel umfasst, die dazu ausgestaltet sind, die Messmittel zu aktivieren, die N Positionen oder N Trajektorien besitzen, die die $S_{opt}$ am nächsten kommende räumliche Verteilung aufweisen.

10. System nach einem der Ansprüche 1 bis 9, wobei der Beobachtungsbereich (1) eine Anzahl M von erlaubten Positionen oder eine Anzahl M von erlaubten Trajektorien umfasst, wobei M>N gilt, wobei den Messmitteln jeweils erlaubt ist, Positionen oder Trajektorien zu besitzen.

11. System nach einem der Ansprüche 1 bis 10, wobei die Messmittel mindestens N Messvorrichtungen umfassen, mit denen jeweils ein Fahrzeug ausgestattet ist, wobei das Fahrzeug bevorzugt ein Straßen- oder Luftfahrzeug ist.

12. System nach einem der Ansprüche 1 bis 10, wobei die Messmittel mindestens N Messvorrichtungen umfassen, mit denen jeweils ein Stadtmöbel ausgestattet ist, wobei das Stadtmöbel bevorzugt aus der Gruppe ausgewählt ist, die Verkehrsampeln und Buswartehäuschen umfasst.

13. System nach einem der Ansprüche 1 bis 12, wobei die Messmittel spektroskopische Sensoren umfassen, bevorzugt nichtdispersive Infrarotsensoren.

14. Verfahren zum Messen mindestens einer physikalischen Größe, die für die Qualität der Luft in einem Beobachtungsbereich (1) repräsentativ ist, wobei das Verfahren die folgenden Schritte umfasst:

> a) Bereitstellen einer Kartierung (2) des Beobachtungsbereichs (1), umfassend eine als V bezeichnete Menge von modellierten Werten, die für die physikalische Größe repräsentativ sind;
> b) Bereitstellen von Messmitteln zum Messen der physikalischen Größe, die eine Anzahl N von Positionen oder eine Anzahl N von Trajektorien in dem Beobachtungsbereich (1) besitzen, wobei die N Positionen oder die N Trajektorien dazu bestimmt sind, eine als $S_{opt}$ bezeichnete räumliche Verteilung aufzuweisen;
> c) Erstellen eines Netzes (3) des Beobachtungsbereichs (1), wobei das Netz (3) eine Anzahl G von Punkten umfasst;
> d) Berechnen, für eine als S bezeichnete gegebene räumliche Verteilung der N Positionen oder der N Trajektorien, eines als $\hat{V}$ bezeichneten Schätzers der Menge V für jeden der G Punkte des Netzes (3) nach der Formel:

$$\hat{V}_j = \frac{\sum_{i=1}^{N} V_i \, w_{ij}}{\sum_{i=1}^{N} w_{ij}}, j \in [\![1, G]\!]$$

> worin:

> - $V_i$ die modellierten Werte der Menge V sind, die für die Positionen oder für die Trajektorien der Messmittel extrahiert werden, die die gegebene räumliche Verteilung S aufweisen,
> - $w_{ij}$ eine Übertragungsfunktion zwischen $s_i$ und $m_j$ ist, wobei $s_i$ die Positionen oder die Trajektorien der Messmittel sind, die die gegebene räumliche Verteilung S aufweisen, und wobei $m_j$ die Positionen der Punkte des Netzes (3) in dem Beobachtungsbereich (1) sind;

> e) Berechnen einer als $\varphi(S)$ bezeichneten Kostenfunktion, die für die Differenz oder die Likelihood zwischen $\hat{V}$ und den als $\overline{V}$ bezeichneten modellierten Werten der Menge V repräsentativ ist, die an den G Punkten des Netzes (3) extrahiert werden;
> f) Extrahieren der räumlichen Verteilung $S_{opt}$, die die Kostenfunktion minimiert oder maximiert, je nachdem, ob die Kostenfunktion für die Differenz oder die Likelihood zwischen $\hat{V}$ und $\overline{V}$ repräsentativ ist;
> g) Anordnen der Messmittel in dem Beobachtungsbereich (1) derart, dass die N Positionen oder die N Trajektorien die räumliche Verteilung $S_{opt}$ aufweisen.

**Claims**

1. System for measuring at least one physical quantity representative of the quality of the air in an observation zone (1), the system comprising:

> - a mapping (2) of the observation zone (1), comprising a set, denoted V, of modelled values representative of the physical quantity;
> - means for measuring the physical quantity, possessing a number N of positions or a number N of trajectories in the observation zone (1), the N positions or the N trajectories being intended to exhibit a spatial distribution, denoted $S_{opt}$, in the observation zone (1);
> - means for calculating the spatial distribution $S_{opt}$, configured to:

> > • construct a mesh (3) of the observation zone (1), the mesh (3) comprising a number G of points;

• calculate, for a given spatial distribution, denoted S, of the N positions or of the N trajectories, an estimator of the set V, denoted $\hat{V}$, for each of the G points of the mesh (3) according to the formula:

$$\hat{V}_j = \frac{\sum_{i=1}^{N} V_i\, w_{ij}}{\sum_{i=1}^{N} w_{ij}}, j \in [\![1, G]\!]$$

where:

- $V_i$ are the modelled values of the set V which are extracted for the positions or for the trajectories of the measurement means exhibiting the given spatial distribution S,
- $w_{ij}$ is a transfer function going between $s_i$ and $m_j$, where $s_i$ are the positions or the trajectories of the measurement means exhibiting the given spatial distribution S, and where $m_j$ are the positions of the points of the mesh (3) in the observation zone (1);
• calculate a cost function, denoted $\varphi(S)$, representative of the difference or of the likelihood between $\hat{V}$ and the modelled values, denoted $\overline{V}$, of the set V which are extracted at the G points of the mesh;
• extract the spatial distribution $S_{opt}$ which minimizes or maximizes the cost function depending on whether the cost function is representative of the difference or of the likelihood between $\hat{V}$ and $\overline{V}$.

2. System according to Claim 1, in which the calculation means are configured to calculate the estimator of the set V according to the formula:

$$\hat{V}_j = \frac{\sum_{i=1}^{N} V_i\, [d(m_j, s_i)]^{-2}}{\sum_{i=1}^{N} [d(m_j, s_i)]^{-2}}, j \in [\![1, G]\!]$$

where:

- $m_j$ are the positions of the points of the mesh (3) in the observation zone (1),
- $s_i$ are the positions or the trajectories of the measurement means exhibiting the given spatial distribution S,
- $V_i$ are the modelled values of the set V which are extracted for the positions or for the trajectories $s_i$,
- d is a distance between a position of a point of the mesh (3) and a position or a trajectory of the measurement means in the observation zone (1).

3. System according to Claim 1 or 2, in which the cost function is representative of the difference between $\hat{V}$ and $\overline{V}$, and the cost function is a norm of the difference between $\hat{V}$ and $\overline{V}$.

4. System according to one of Claims 1 to 3, in which the cost function is representative of the difference between $\hat{V}$ and $\overline{V}$, and in which the calculation means are configured to calculate the cost function according to the formula:

$$\varphi(S) = \frac{1}{G} \sum_{j=1}^{G} (\hat{V}_j - \bar{V}_j)^2$$

where $\overline{V}_j$ are the modelled values of the set V which are extracted for the points of the mesh (3).

5. System according to one of Claims 1 to 4, in which the cost function is representative of the difference between $\hat{V}$ and $\overline{V}$, and in which the calculation means are configured to perform a random modification of the given spatial distribution S according to a probability, denoted $p_{ji}$, satisfying:

$$p_{ji} = 1 \; if \; \varphi(S^{(j)}) \leq \varphi(S^{(i)})$$

$$p_{ji} = exp\left(\frac{\varphi(S^{(i)}) - \varphi(S^{(j)})}{c}\right) if \; \varphi(S^{(j)}) > \varphi(S^{(i)})$$

where:

- $S^{(i)}$ is an initial given spatial distribution,
- $S^{(j)}$ is a given spatial distribution randomly modified,
- c is a parameter;

and in which the calculation means are configured to iterate the random modification of the given spatial distribution S until the extraction of $S_{opt}$.

6. System according to Claim 1 or 2, in which the cost function is representative of the likelihood between $\hat{V}$ and $\overline{V}$, and the cost function comprises a scalar product of $\hat{V}$ and $\overline{V}$, preferably normalized.

7. System according to Claim 1 or 2 or according to Claim 6, in which the cost function is representative of the likelihood between $\hat{V}$ and $\overline{V}$, and in which the calculation means are configured to perform a random modification of the given spatial distribution S according to a probability, denoted $p_{ji}$, satisfying:

$$p_{ji} = 1 \; if \; \varphi(S^{(j)}) \geq \varphi(S^{(i)})$$

$$p_{ji} = exp\left(\frac{\varphi(S^{(j)}) - \varphi(S^{(i)})}{c}\right) if \; \varphi(S^{(j)}) < \varphi(S^{(i)})$$

where:

- $S^{(i)}$ is an initial given spatial distribution,
- $S^{(j)}$ is a given spatial distribution randomly modified,
- c is a parameter;

and in which the calculation means are configured to iterate the random modification of the given spatial distribution S until the extraction of $S_{opt}$.

8. System according to one of Claims 1 to 7, in which the measurement means are arranged in the observation zone (1) in such a way as to possess N positions or N trajectories exhibiting the spatial distribution $S_{opt}$.

9. System according to one of Claims 1 to 7, comprising activation means configured to activate the measurement means possessing N positions or N trajectories exhibiting the spatial distribution which is closest to $S_{opt}$.

10. System according to one of Claims 1 to 9, in which the observation zone (1) comprises a number M of permitted positions or a number M of permitted trajectories, satisfying M>N, in which the measurement means are respectively permitted to possess positions or trajectories.

11. System according to one of Claims 1 to 10, in which the measurement means comprise at least N measurement devices each fitted to a vehicle, the vehicle preferably being a road vehicle or aerial vehicle.

12. System according to one of Claims 1 to 10, in which the measurement means comprise at least N measurement devices each fitted to an item of urban furniture, the urban furniture preferably being selected from the group comprising traffic lights and bus shelters.

13. System according to one of Claims 1 to 12, in which the measurement means comprise spectroscopic sensors, preferably non-dispersive infrared sensors.

**14.** Method for measuring at least one physical quantity representative of the quality of the air in an observation zone (1), the method comprising the steps:

a) provide a mapping (2) of the observation zone (1), comprising a set, denoted V, of modelled values representative of the physical quantity;
b) provide means for measuring the physical quantity, possessing a number N of positions or a number N of trajectories in the observation zone (1), the N positions or the N trajectories being intended to exhibit a spatial distribution, denoted $S_{opt}$;
c) construct a mesh (3) of the observation zone (1), the mesh (3) comprising a number G of points;
d) calculate, for a given spatial distribution, denoted S, of the N positions or of the N trajectories, an estimator of the set V, denoted $\hat{V}$, for each of the G points of the mesh (3) according to the formula:

$$\widehat{V}_{\mathrm{j}} = \frac{\sum_{i=1}^{N} V_i\, w_{ij}}{\sum_{i=1}^{N} w_{ij}}, \mathrm{j} \in [\![1, G]\!]$$

where:

- $V_i$ are the modelled values of the set V which are extracted for the positions or for the trajectories of the measurement means exhibiting the given spatial distribution S,
- $w_{ij}$ is a transfer function going between $s_i$ and $m_j$, where $s_i$ are the positions or the trajectories of the measurement means exhibiting the given spatial distribution S, and where $m_j$ are the positions of the points of the mesh (3) in the observation zone (1);

e) calculate a cost function, denoted $\varphi(S)$, representative of the difference or of the likelihood between $\hat{V}$ and the modelled values, denoted $\overline{V}$, of the set V which are extracted at the G points of the mesh (3);
f) extract the spatial distribution $S_{opt}$ which minimizes or maximizes the cost function depending on whether the cost function is representative of the difference or of the likelihood between $\hat{V}$ and $\overline{V}$;
g) arrange the measurement means in the observation zone (1) so that the N positions or the N trajectories exhibit the spatial distribution $S_{opt}$.

Fig. 1

*Fig. 2*

*Fig. 3*

*Fig. 4*

Fig. 5

Fig. 6

*Fig. 7*

*Fig. 8*

Fig. 9

Fig. 10

*Fig. 11*

*Fig. 12*

*Fig. 13*

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- Guidelines for Ambient Air Quality Monitoring. CPCB (Central Pollution Control Board), Avril 2003
- **CASELTON ; ZIDEK.** Optimal Monitoring Network design. *Statistics & Probability Letters,* 1984, vol. 2, 223-227
- **R. BERKOWICZ et al.** Modeling trafic pollution in streets. National Environmental Research Institute, Janvier 1997
- **R. BERKOWICZ.** OSPM - A Parameterised Street Pollution Model. *Environmental Monitoring and Assessment,* 2000, vol. 65 (1), 323-331